# EUROPEAN PATENT APPLICATION

(11) **EP 2 484 353 A1**
(43) Date of publication of application: **08.08.2012**
(21) Application number: 10820190.6
(22) Date of filing: 31.03.2010
(51) Int. Cl.: A61K 31/198, A23L 1/305, A61K 8/44, A61P 17/00, A61P 17/16, A61P 27/12, A61P 29/00, A61P 31/22, A61P 35/00, A61P 37/08, A61Q 17/04, A61Q 19/08

(54) **ORAL COMPOSITION FOR ALLEVIATION OF ULTRAVIOLET RADIATION-INDUCED DAMAGE**

(30) Priority: 30.09.2009 JP 2009228267
(71) Applicant: SHISEIDO COMPANY, LTD., Tokyo 104-8010 (JP)
(72) Inventor: ASHIDA, Yutaka, Yokohama-shi Kanagawa 236-0004 (JP); TOJO, Yosuke, Yokohama-shi Kanagawa 236-0004 (JP); MIZUMOTO, Chieko, Yokohama-shi Kanagawa 236-0004 (JP); MITA, Masashi, Tokyo 104-8010 (JP); SHIMADA, Shoichiro, Yokohama-shi Kanagawa 236-0004 (JP)
(74) Representative: Henkel, Breuer & Partner
(86) International application number: PCT/JP2010/055841
(87) International publication number: WO 2011/040070

(57) **Abstract**

Disclosed are: an oral composition for alleviating ultraviolet radiation-induced damage, which can be used on a daily basis and is stable and safe; and a medicinal agent, an anti-wrinkle agent and a food composition, each of which comprises the oral composition. Specifically disclosed is an oral composition for alleviating ultraviolet radiation-induced damage, which comprises at least one compound selected from the group consisting of methionine and derivatives and/or salts thereof. The composition may be an anti-wrinkle agent, a pharmaceutical composition for treating and/or preventing skin diseases, a food composition, or a pharmaceutical composition for preventing or treating cataract. In the oral composition for alleviating ultraviolet radiation-induced damage, methionine may have a D-form.

## Description

### TECHNICAL FIELD

The present invention relates to an oral composition for alleviating ultraviolet irradiation-induced damage comprising one or more compounds selected from a group consisting of methionine and derivatives and/or salts thereof, a method for improving an ultraviolet irradiation exposure-induced skin disease and an cosmetic skin condition comprising a step of administering one or more compounds selected from the group consisting of methionine and derivatives and/or salts thereof, and a method for treating and/or preventing cataract comprising a step of administering one or more compounds described above.

### Background Art

Ultraviolet rays are classified into long wavelength region ultraviolet rays of longer than about 320 nm (UV-A), medium wavelength region ultraviolet rays of about 320 to about 280 nm (UV-B) and short wavelength region ultraviolet rays of shorter than about 280 nm (UV-C). Among these, the UV-C is not contained in solar lights reaching the ground since it is absorbed by the ozone layer. The UV-A is not absorbed by the ozone layer and predominant in the ultraviolet rays reaching the ground. While the UV-B is partly absorbed by the ozone layer, it causes a skin damage at one-thousandth dose of the UV-A. Accordingly, both of the UV-A and the UV-B are important as major causes of the skin damages. Non-Patent Literature 1 discloses diseases in which the ultraviolet rays are implicated, including wrinkles, erythema, xeroderma pigmentosum, chronic actinic dermatitis, squamous cell carcinoma, basal cell carcinoma, malignant melanoma, Bowen disease, solar keratosis, photodermatosis, hydroa vacciniforme and photocontact dermatitis, while Non-Patent Literature 2 exemplifies solar dermatitis, chronic actinic dermatopathy, actinic keratosis, actinic cheilitis, Favre-Racouchot syndrome, photodermatosis, photocontact dermatitis, berloque dermatitis, photosensitive drug eruption, polymorphous light eruption, hydroa vacciniforme, solar urticaria, chronic photosensitive dermatitis, xeroderma pigmentosum, ephelides, porphyria, pellagra, Hartnup disease, solar keratosis, dermatomyositis, lichen planus, Darier disease, pityriasis rubra pilaris, rosacea, atopic dermatitis, chloasma, prurigo simplex and lupus erythematosus.

### PRIOR ART DOCUMENTS

### Non-Patent Documents

Non-Patent document 1: "HIFUSHIKKAN SAISHIN NO CHIRYO (Latest methods for treating dermal diseases) ", 2005-2006 (Nankodo Co., Ltd.)
Non-Patent document 2: "HYOJUN HIFUKAGAKU (Standard dermatology)", 7th edition (Igaku-Shoin Ltd.)

### Disclosure of the Invention

### PROBLEM TO BE SOLVED BY THE INVENTION

Known conventional agents for preventing and/or treating an ultraviolet irradiation-induced skin damage include an ultraviolet scattering agent which inhibits absorption of the ultraviolet by the skin, such as titanium oxide, an ultraviolet absorber, such as ethyl hexyl p-methoxycinnamic acid, or an antioxidant which scavenge a free radical generated by the ultraviolet. The ultraviolet scattering agent or the ultraviolet absorber is, however, not used on a daily basis although it is effective outdoor in preventing sunburn. The antioxidant is problematic in terms of stability and safety. In addition, known therapeutic agents for the ultraviolet irradiation-induced skin damages are limited only to symptomatic therapeutic agents. On the other hand, use of an external preparation for the skin has a difficulty in a continuous administration. Accordingly, it is required to develop an oral composition for alleviating ultraviolet irradiation-induced damage, an anti-wrinkle agent, a pharmaceutical composition for treating and/or preventing skin diseases, a food composition, or a pharmaceutical composition for preventing or treating cataract, which can be used on a daily basis and ingested orally, and is stable and safe.

### MEANS FOR SOLVING THE PROBLEM

The present invention provides an oral composition for alleviating an ultraviolet irradiation-induced damage comprising one or more compounds selected from the group consisting of methionine and derivatives and/or salts thereof.

In the oral composition for alleviating ultraviolet irradiation-induced damage according to the present invention, the methionine described above may be in the D-form.

The oral composition for alleviating ultraviolet irradiation-induced damage described above may be an anti-wrinkle agent.

The oral composition for alleviating ultraviolet irradiation-induced damage described above may be used as a pharmaceutical product for skin diseases.

The skin disease described above may be selected from the group consisting of erythema, solar dermatitis, chronic actinic dermatopathy, actinic keratosis, actinic cheilitis, Favre-Racouchot disease, photodermatosis, photocontact dermatitis, berloque dermatitis, photosensitive drug eruption, polymorphous light eruption, hydroa vacciniforme, solar urticaria, chronic photosensitive dermatitis, xeroderma pigmentosum, ephelides, porphyria, pellagra, Hartnup disease, solar keratosis, dermatomyositis, lichen planus, Darier disease, pityriasis rubra pilaris, rosacea, atopic dermatitis, chloasma, prurigo simplex, lupus erythematosus, squamous cell carcinoma, basal cell carcinoma and Bowen disease.

In the oral composition for alleviating an ultraviolet irradiation-induced damage according to the present invention, the pharmaceutical product for skin disease described above may be an agent for treating skin disease.

In the oral composition for alleviating an ultraviolet irradiation-induced damage according to the present invention, the pharmaceutical product for skin disease described above may be an agent for preventing skin disease.

The oral composition for alleviating an ultraviolet irradiation-induced damage according to the present invention may be used as a food product.

The oral composition for alleviating an ultraviolet irradiation-induced damage according to the present invention may be used as a pharmaceutical product for cataract.

In the oral composition for alleviating an ultraviolet irradiation-induced damage according to the present invention, the pharmaceutical product for cataract described above may be an agent for treating or preventing cataract.

The cataract described above may be senile cataract.

The present invention provides a method for treating and/or preventing an ultraviolet irradiation exposure-induced skin disease, comprising a step of administering an oral composition for alleviating ultraviolet irradiation-induced damage, wherein the composition is comprised of one or more compounds selected from a group consisting of methionine and derivatives and/or salts thereof. The skin disease described above may be selected from the group consisting of erythema, solar dermatitis, chronic actinic dermatopathy, actinic keratosis, actinic cheilitis, Favre-Racouchot disease, photodermatosis, photocontact dermatitis, berloque dermatitis, photosensitive drug eruption, polymorphous light eruption, hydroa vacciniforme, solar urticaria, chronic photosensitive dermatitis, xeroderma pigmentosum, ephelides, porphyria, pellagra, Hartnup disease, solar keratosis, dermatomyositis, lichen planus, Darier disease, pityriasis rubra pilaris, rosacea, atopic dermatitis, chloasma, prurigo simplex, lupus erythematosus, squamous cell carcinoma, basal cell carcinoma and Bowen disease. The methionine described above may be in the D-form.

The present invention provides a method for improving a cosmetic skin condition, comprising a step of administering an oral composition for alleviating ultraviolet irradiation-induced damage, wherein the composition is comprised of one or more compounds selected from the group consisting of methionine and derivatives and/or salts thereof. In the method for improving the cosmetic skin condition described above, the oral composition for alleviating ultraviolet irradiation-induced damage comprising one or more compounds selected from the group consisting of methionine and derivatives and/or salts thereof described above may be a food composition. The methionine described above may be in the D-form.

In the method for improving the cosmetic skin condition according to the present invention, the improvement of the cosmetic skin condition includes, but is not limited to, an anti-wrinkle treatment.

The present invention may provide a method for treating and/or preventing cataract, comprising a step of administering a composition, wherein the composition is comprised of one or more compounds selected from the group consisting of L- or D-methionine and derivatives and/or salts thereof.

In the method for treating and/or preventing cataract according to the present invention, the cataract described above may be senile cataract.

As used herein, "salt" of methionine refers to any salt including metal salts, amine salts, etc., provided that the ultraviolet irradiation-induced damage alleviating effect of methionine is not impaired. The metal salt described above may include an alkaline metal salt, an alkaline earth metal salt and the like. The amine salt described above may include a triethylamine salt, a benzylamine salt and the like.

As used herein, "derivative" of methionine refers to a methionine molecule bound covalently to any substituent group at its amino group, carboxyl group or side chain, provided that the alleviating effect of methionine on ultraviolet irradiation-induced damage is not impaired. The substituent group described above includes, but is not limited to, a protective group, such as an N-phenylacetyl group, a 4, 4'-dimethoxytrityl (DMT) group, etc.; a biological macromolecule, such as a protein, a peptide, a saccharide, a lipid, a nucleic acid, etc.; a synthetic polymer, such as a polystyrene, a polyethylene, a polyvinyl, a polyester, etc.; and a functional group such as an ester group, etc. The ester group mentioned above may include, for example, a methyl ester, an ethyl ester, other aliphatic ester or aromatic ester.

Since an amino acid exists as an optical isomer which is in either of an L-form or a D-form but a natural protein is made of L-amino acids bound via peptide bonds and only L-amino acids are employed excluding some exceptions such as a bacterial cell wall, it has been considered that in a mammal including a human only L-amino acids are present and utilized (Kinouchi, T. et al., TANPAKUSHITSU KAKUSAN KOSO (PROTEIN, NUCLEIC ACID AND ENZYME), 50:453-460 (2005), Lehninger Principles of Biochemistry [Vol.1] 2nd ed., pp132-147 (1993), Japanese-language translation, Hirokawa Publishing Co., Harper's Biochemistry, Original version, 22nd ed., pp21-30(1991), Japanese-language translation, Maruzen Co., Ltd.). Accordingly, only L-amino acids have exclusively been employed as amino acids academically and industrially for a long time.

Exceptional cases where a D-amino acid is employed include, for example, a case of using as a raw material for an antibiotics produced by a microorganism and a case of a food additive employing a D-amino acid as it is as a DL-amino acid mixture for the purpose of reducing cost of fractionating only an L-amino acid from a mixture of the equivalent amounts of Land D-amino acids produced through a chemical synthesis of amino acids. However, there has been no case of industrially utilizing an L-amino acid-free D-amino acid alone as a bioactive substance.

D-Serine and D-aspartic acid have been studied to a comparatively advanced stage because of their higher ratios of D-forms. D-Serine is localized in a cerebrum and a hippocampus, and is known to be involved in an NMDA receptor modulator in the brain. D-Aspartic acid is demonstrated to be localized in a testis and a pineal body, and to be involved in the regulation of hormone secretion (Japanese Patent Unexamined Publication No.2005-3558). However, the physiological effects of L- and D-methionine on the skin, especially on an ultraviolet irradiation-induced damage of the skin has not been elucidated.

As indicated in Examples described below, methionine has not been known so far to have an alleviating effect of methionine as being L-form or D-form, or a mixture thereof, on ultraviolet irradiation-induced damage. Therefore, an oral composition for alleviating an ultraviolet irradiation-induced damage comprising L- and/or D-methionine according to the present invention is a novel invention.

Recently, it was reported that ddY mice had been allowed to access a 10 mM aqueous solution of a D-amino acid for 2 weeks and then determined for the D-amino acid concentration in each organ, which was 3 to 1000 pmol per gland in a pineal body and 2 to 500 nmol per wet gram in a brain tissue (Morikawa, A. et al., Amino Acids, 32:13-20 (2007)). Based on this report, the lower limit of daily dose of L- and D-methionine contained in a composition of the present invention was calculated.

As indicated in Examples described below, methionine relevant to the present invention exhibits an alleviating effect on ultraviolet irradiation-induced damage at a concentration ranging from 0.001 to 100 µM (micro-molar) on a cultured human fibroblast or a concentration of 10 mM on mice. Accordingly, the amount of methionine contained in a pharmaceutical composition of the present invention, anti-wrinkle agent and food composition may vary in a wide range, provided that methionine is delivered to a fibroblast in an in vivo skin tissue at a concentration range specified above. When the composition of the present invention is an internal preparation, the methionine content may be 0.000001% by weight to 100% by weight. When the composition of the present invention is an internal preparation, the methionine content is preferably 0.000002% by weight to 80% by weight, most preferably 0.00002% by weight to 60% by weight. The lower limit of the daily dose of D-methionine contained in the composition of the present invention may be 0.01 ng, preferably 0.1 ng, more preferably 1 ng per kg body weight. The lower limit of the daily dose of L-methionine acid contained in the composition of the present invention is less than a dose of a clinical drug (2 mg or more per kg body weight), and may be 0.01 mg, preferably 0.1 mg, more preferably 1 mg per kg body weight.

The pharmaceutical composition of the present invention may further comprise one or more pharmaceutically acceptable additives, in addition to methionine alone , methionine, salts and/or derivatives capable of being subjected to drug metabolizing enzymes and the like in vivo whereby releasing methionine, provided that the alleviating effect of methionine on ultraviolet irradiation-induced damage of methionine is not impaired. Such an additive includes, but is not limited to, a diluent, a swelling agent, a binder, an adhesive, a lubricant, a glidant, a plasticizer, a disintegrant, a carrier solvent, a buffering agent, a colorant, a flavoring agent, a sweetener, a preservative, a stabilizer, an adsorbent, as well as other pharmaceutical additives known to those skilled in the art.

An anti-wrinkle agent of the present invention can be prepared using only methionine, salts of methionine and/or derivatives capable of being subjected to in vivo drug metabolizing enzymes and the like whereby releasing methionine. However, other components employed in external preparations for skin such as cosmetic and pharmaceutical products including quasi drugs may appropriately be incorporated as required to the extent that the effect of the invention is not impaired. Such other components (optionally incorporated components) include, for example, oils, surfactants, powders, colorants, water, alcohols, thickening agents, chelating agents, silicones, antioxidants, UV absorbers, humectants, flavoring agents, various medicinal ingredients, preservatives, pH adjusters, neutralizers.

The food composition of the present invention may further comprise, in addition to methionine, salts of methionine and/or derivatives capable of being subjected to in vivo drug metabolizing enzymes and the like whereby releasing methionine, a food-industrially acceptable component, such as a seasoning, a colorant, a preservative, provided that the alleviating effect of methionine on ultraviolet irradiation-induced damage is not impaired.

The food composition of the present invention may be any of those employed conventionally in a food composition, such as a beverage, a gummy candy, a candy, a tablet sweet, to which it is not limited.

An exposure to an ultraviolet ray is considered to be one of the causes not only of a skin disease but also of an ophthalmic disease such as cataract. A prolonged exposure of mice to an ultraviolet ray was reported to cause a turbidity in an anterior cortex of the lens, whereby allowing a cataract model to be obtained experimentally (Maeda, T. and Iwata, S., "SUISHOTAI SONO SEIKAGAKUTEKI KIKO (Lens, its biochemical mechanisms), pp.318-323, Ed. by Iwata, S., Medical-Aoi Publishings, Inc., Tokyo (1986)). Also, one of the causes of senile cataract is considered to be an ultraviolet ray (Fujinaga, Y., "HAKUNAISHO GANKA (cataract ophthalmology) MOOK No.17", p.10, Ed. by Mishima et al. , Kanehara & Co., Ltd. , Tokyo (1982)), and Zigman et al. conducted an epidemiological surveillance in Manila, Tampa and Rochester and reported that there was a correlation between the ultraviolet irradiation dose and the cataract incidence and the ultraviolet ray is a risk factor for the cataract (Zigman, S. et al., Invest. Ophthalmol. Visual Sci. 18:462-467(1979)). Accordingly, these findings, in combination with Examples described below, suggest that L- and D-methionine having an alleviating effect on ultraviolet irradiation-induced damage are effective in preventing or treating cataract.

### BRIEF DESCRIPTION OF THE DRAWINGS

Figure 1 is a graph showing the effect of L- or D-methionine addition before an ultraviolet irradiation in normal human dermal fibroblasts.
Figure 2 is a graph showing the effect of D-methionine, addition after an ultraviolet irradiation in normal human dermal fibroblasts.
Figure 3 is a graph showing the effect of D-methionine, administration on increase in skin thickness in ultraviolet-irradiated mice.
Figure 4 is a graph showing the effect of D-methionine administration on wrinkling in ultraviolet-irradiated mice.
Figure 5 is a graph showing the effect of L- or D-methionine administration on wrinkling in ultraviolet-irradiated mice.

### DESCRIPTION OF EMBODIMENTS

Examples of the present invention described below are intended only to exemplify the invention rather than to limit the scope thereof. The scope of the invention is limited only by the description in claims.

The following investigation was made after obtaining an approval by Ethical Committee of Shiseido Research Center in compliance with a guideline by National Institutes of Health (NIH) in United States.

### Example 1

### Ultraviolet irradiation-induced damage test in cultured cell Ultraviolet irradiation and methionine addition method

The cell employed was a commercially available human neonatal dermal fibroblast (Cryo NHDF-Neo, Sanko-Junyaku Co., Ltd.). This cell was inoculated at 2 × 10⁵ cells/mL to a commercially available culture dish of 35 mm in diameter (BD FALCON 353001, Becton Dickinson Japan), where it was cultured in a commercially available cell culture medium (D-MEM (1 g/L glucose, Wako Pure Chemical Industries, Ltd.) supplemented with 10% fetal bovine serum and 1% antibiotic (15240-062, GIBCO) (hereinafter, referred to as "ordinary medium"). This cell was cultured for about 24 hours in a 5% CO₂ and saturated water vapor atmosphere at 37°C(degrees Celcius).

Thereafter, the culture medium for culturing the cell described above was switched to 1 mL of a BSO medium supplemented with a glutathione biosynthesis inhibitor BSO (L-buthionine-(S,R)-sulfoximine, Wako Pure Chemical Industries, Ltd.) at 1 × 10⁻³%, where the culture was conducted for about 24 hours in a 5% CO₂ and saturated water vapor atmosphere at 37°C (degrees Celcius). The BSO medium described above was prepared by a 200-fold dilution of a stock solution containing 0.2% BSO dissolved in ethyl alcohol with the ordinary medium described above.

### Addition of methionine before ultraviolet irradiation

For determining the effect of adding methionine before ultraviolet irradiation (hereinafter, referred to as "pre-irradiation addition"), the culture medium was switched to a BSO medium supplemented with 0.001 to 100 µM (micro-molar) L- or D-methionine 24 hours before the irradiation. The ultraviolet irradiation after switching into a medium added with 0.1 µM (micro-molar) D-proline was employed as a positive control, while the ultraviolet irradiation to the medium still being free of such an added amino acid was employed as a negative control.

### UV Irradiation medium

Ferric chloride (II) was dissolved in a distilled water at 2 x 10⁻³%, and the resultant solution was subjected to a 200-fold dilution (final concentration: 1 × 10⁻⁵%) with a phosphate buffer solution PBS (+) containing calcium ion and magnesium ion to obtain a medium (hereinafter, referred to as "UV irradiation medium"), which was warmed preliminarily to 37°C before use.

### UV Irradiation

Before UV-A irradiation, the culture medium was replaced with 1 mL of the UV irradiation medium described above. The UV-A irradiation was conducted using a UV light uniform exposure device UVE-502S+EL-160 (SAN-EI ELECTRIC) by irradiating a UV ray of 320 nm to 400 nm at 8 J/cm² or 9 J/cm² from about 20 cm above a culture dish in a state where the lid of the respective culture dish was removed. The UV dose was measured using UV RADIOMETER UVR-3036/S (Topcon Corporation).

### Addition of methionine after ultraviolet irradiation

After the ultraviolet irradiation, the medium was switched back to the ordinary medium described above, and the culture was conducted in a 5% CO₂ and saturated water vapor atmosphere at 37°C (degrees Celcius) for 40 hours. For determining the effect of adding methionine after UV irradiation (hereinafter, referred to as "post-irradiation addiction"), this 40-hour cultured medium was supplemented with 0.001 to 100 µM (micro-molar) L- or D-methionine.

### Cell damage quantification of pre- and post-irradiation additions

Thereafter, the culture medium was supplemented with Alamar Blue (trade mark, Invitrogen) at a final concentration of 10%, and its supernatant was determined for the fluorescent intensity three hours later with an excitation wavelength of 544 nm and a fluorescent wavelength of 590 nm as described by Ahmed S.A. et al. (J. Immunol. Method. 170, 211-224 (1994)) and in accordance with the manufacture's instruction.

### Results of pre-irradiation addition

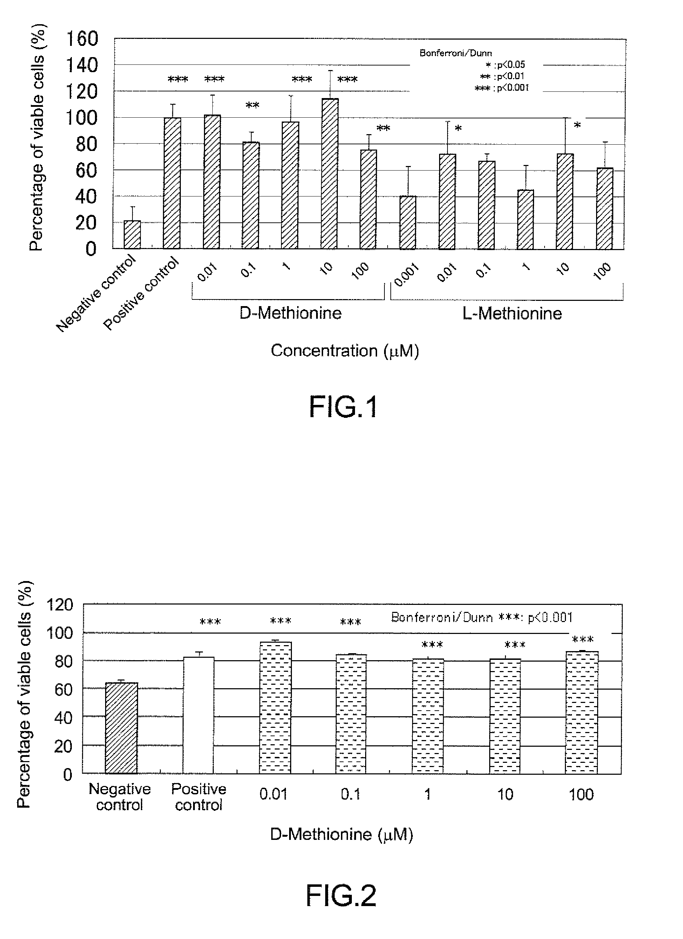

Figure 1 shows the results of the experiment investigating the effect of the pre-irradiation addition of L- or D-methionine on the damage in the fibroblast induced by the ultraviolet irradiation with the UV-A at 9 J/cm². The error bars for relevant experimental conditions are the standard deviations of the measured values of the results of the experiments repeated four times under same conditions. The asterisk (*) indicates P<5%, asterisk (**) indicates P<1% and asterisk (***) indicates P<0.1%, all in Bonferroni/Dunn test.

The percentage of the viable cell in the absence of the added amino acid before the UV-A 9 J/cm² irradiation (negative control) was 24%. The percentage of the viable cell in the presence of the added D-proline at 0.1 µM (micro-molar) before the irradiation (positive control) was 100%, showing the alleviated cell damage. Pre-irradiation addition of D-methionine at 0.01 µM(micro-molar), 0.1 µM(micro-molar), 1 µM(micro-molar) , 10 µM(micro-molar) or 100 µM(micro-molar) resulted in the percentage of the viable cell of 102%, 81%, 97%, 114% or 76%, respectively. Pre-irradiation addition of L-methionine at 0.001 µM(micro-molar), 0.01 µM(micro-molar), 0.1 µM(micro-molar) 1 µM (micro-molar), 10 µM(micro-molar) or 100 µM(micro-molar) resulted in the percentage of the viable cell of 40%, 72%, 67%, 45%, 73% or 62%, respectively. Based on these results, the addition of L- or D-methionine resulted in an increased percentage of the viable cell and a reduced cell death.

### Results of post-irradiation addition

Figure 2 shows the results of the experiment investigating the effect of the post-irradiation addition of D-methionine on the damage of the fibroblast induced by the ultraviolet irradiation with the UV-A at 8 J/cm². The error bars for relevant experimental conditions are the standard deviations of the measured values of the results of the experiments repeated four times under same conditions. The asterisk (***) indicates P<0.1% in Bonferroni/Dunn test.

The percentage of the viable cell in the absence of the added amino acid after the UV-A 8 J/cm² irradiation (negative control) was 64%. The percentage of the viable cell in the presence of the added D-methionine at 0.1 µM (micro-molar) (positive control) was 82%, showing the reduced cell death. Post-irradiation addition of D-methionine at 0.01 µM(micro-molar), 0.1 µM(micro-molar), 1 µM(micro-molar), 10 µM(mi cro-mol ar) or 100 µM(micro-molar) resulted in the percentage of the viable cell of 93%, 84%, 82%, 81% or 87%, respectively. Based on these results, the addition of D-methionine resulted in an increased percentage of the viable cell and a reduced cell death. It was also revealed that the cell death reducing effect was observed regardless of the time whether before or after the UV irradiation the D-methionine was added. In addition, L-methionine also reduced the cell death induced by the UV irradiation. Accordingly, it was suggested that there was no relevancy to the time whether before or after the UV irradiation the L-methionine was added.

### Example 2

### Test of inhibition of UV irradiation-induced damage-promoted wrinkling in hairless mice (1)

### UV irradiation and D-methionine administration methods

Male hairless mice (Hos: HR-1, six-week old, Hoshino Laboratory Animals, Inc.) were employed and subjected to a partly modified method by Schwarz et al. (Haratake A. et al. J. Invest. Dermatol. 108:769-775,1997.) in accordance with the repetitive UV-B irradiation method (Naganuma M. et al. J. Dermatol. Sci. 25:29-35, 2001. Schwartz E. J. Invest. Dermatol. 91:158-161, 1988.) whereby forming wrinkles. Thus, the dorsal region was irradiated with UV-B (light source: Toshiba FL-20 SE fluorescent lamp manufactured by Toshiba Electric) three times a week for 12 weeks. The irradiation dose was initially 36 mJ/cm²/time, which was gradually increased in the second week, and thereafter it was increased to 216 mJ/cm²/time in the 12th week. The total irradiation dose was 4.482 J/cm². The UV dose measured using UVRADIOMETER (UVR-305/365D (II), Topcon Corporation) was employed as a readout. During the UV-B irradiation, 10 mM D-methionine was given via a water supplier bottle. As a control, a tap water was given in the same manner. The water supplier bottle was exchanged once a week.

### Measurement method of UV irradiation-induced damage-promoted skin thickening

After the end of the UV irradiation, the skin thickness was measured using a thickness gauge (Mitsutoyo Corporation, PK-1012SU). The measured value of the skin thickness was designated as "skin thickness x 2", since the measurement was conducted by pinching the skin with the thickness gauge.

### Wrinkling determination method

After the irradiation to the mice described above, the dorsal regions of the mice were photographed, and a partly modified method by Bissett et al. (Bissett DL. et al. Photochemistry and Photobiology 46:367-378,1987.) was employed to evaluate the degree of wrinkling in accordance with the criteria indicated in Table 1 below. The wrinkle evaluation was performed separately by four observers, who thereafter consulted together to determine a score about the wrinkle grade (hereinafter, simply referred to as "score").

### Measurement results of skin thickness

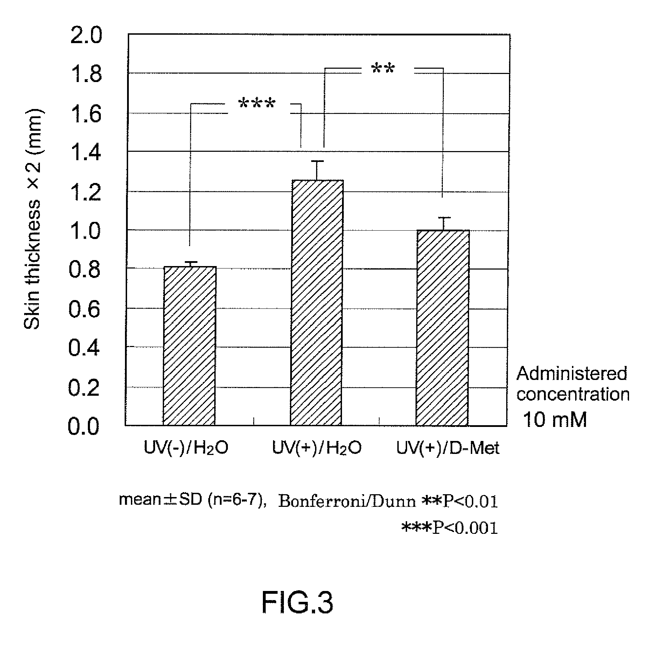

Figure 3 shows the results of the experiment investigating the effect of D-methionine on the skin thickening induced by a UV-B irradiation at a total irradiation dose of 4.482 J/cm². The error bars for relevant experimental conditions are The standard deviations of the measured values of the results of the experiments repeated six or seven times under same conditions. The asterisk (∗∗) indicates P<1% and asterisk (***) indicates P<0.1% in Bonferroni/Dunn test. The measured value of the skin thickness after the UV-B irradiation in the non-UV-B irradiated and non-D-methionine administered group (UV(-)/H₂0) was 0.81 mm and that in the UV-B irradiated and non-D-methionine administered group (UV(+)/H₂0) was 1.25 mm, and that in the UV-B irradiated and D-methionine administered group (UV(+)/D-Met) was 1.00. These results indicated that the UV-B irradiation caused an ultraviolet irradiation-induced damage which resulted in a marked thickening of the skin and that a statistically significant reduction in this skin thickening was achieved by D-methionine.

**[Table 1]**

| Score | Criteria |
|---|---|
| 0 | No wrinkle is observed. |
| 1 | Wrinkles are shallower, shorter or fewer than score 2. |
| 2 | Shallow wrinkles are observed. |
| 3 | Wrinkles are deeper or longer than score 2. |
| | Wrinkles are shallower, shorter or fewer than score 4. |
| 4 | Shallow wrinkles are observed over the entire surface. |
| 5 | Wrinkles are deeper or longer than score 4. |
| | Wrinkles are shallower or shorter than score 6. |
| 6 | Deep and long wrinkles are observed. |
| 7 | There are increased wrinkles that are deeper and longer than score 6. |
| | Wrinkles are shallower or shorter than score 8. |
| 8 | Deep and long wrinkles are observed over the entire surface. |

### Evaluation results of wrinkling

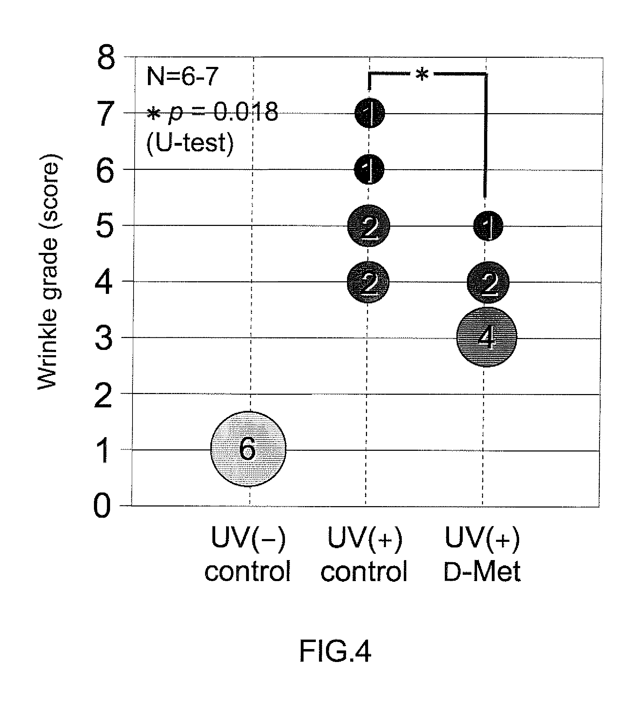

Figure 4 shows the results of the experiment investigating the effect of D-methionine on the wrinkling induced by a UV-B irradiation at a total irradiation dose of 4.482 J/cm². The error bars for relevant experimental conditions are the standard deviations of the measured values of the results of the experiments repeated six or seven times under same conditions. The asterisk (*) indicates P=1.8% in Mann-Whitney U test. The number of animals of each score after the UV-B irradiation was as follows. The non-UV-B irradiated and non-D-methionine administered group (UV(-) control) had six animals scored 1. The UV-B irradiated and non-D-methionine administered group (UV(+) control) had 2 animals scored 4, two animals scored 5, one animal scored 6 and one animal scored 7. The UV-B irradiated and D-methionine administered group (UV(+) D-Met) had four animals scored 3, two animals scored 4 and one animal scored 5. These results indicated that the UV-B irradiation caused an ultraviolet irradiation-induced damage which resulted in a marked increase in the wrinkling and that a statistically significant reduction in this wrinkling was achieved by D-methionine.

### Example 3

### Test of inhibition of UV irradiation-induced damage-promoted wrinkling in hairless mice (2)

### Methods

During the UV-B irradiation, 10 mM L- or D-methionine was given via a water supplier bottle. The UV irradiation and the wrinkling determination were conducted by the methods described in Example 2.

### Results

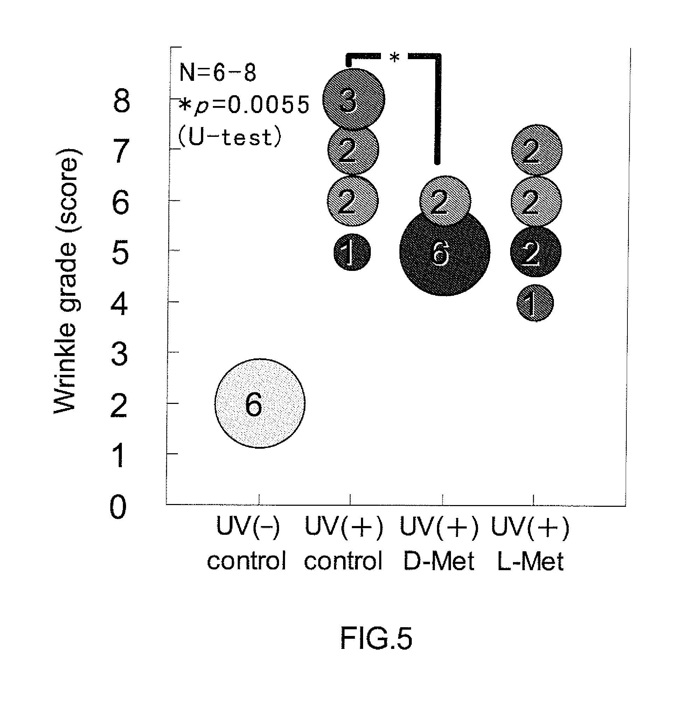

Figure 5 shows the results of the experiment investigating the effects of L- and D-methionine on the wrinkling induced by a UV-B irradiation at a dose corresponding to that in Example 2. The error bars for relevant experimental conditions are the standard deviations of the measured values of the results of the experiments repeated six to eight times under same conditions. The asterisk (*) indicates P=0.55% in Mann-Whitney U test.

The number of animals of each score after the UV-B irradiation was as follows. The non-UV-B irradiated and non-D-methionine administered group (UV(-) control) had six animals scored 2. The UV-B irradiated and non-D-methionine administered group (UV(+) control) had one animal scored 5, two animals scored 6, two animals scored 7 and three animals scored 8. The UV-B irradiated and D-methionine administered group (UV(+) D-Met) had six animals scored 5 and two animals scored 6. The UV-B irradiated and L-methionine administered group (UV(+) L-Met) had one animal scored 4, two animals scored 5, two animals scored 6 and two animals scored 7. These results indicated that the UV-B irradiation caused an ultraviolet irradiation-induced damage which resulted in a marked increase in the wrinkling and that a statistically significant reduction in this wrinkling was achieved by D-methionine. It was also indicated that L-methionine reduced the cell death induced by UV-A irradiation as shown in Example 1 but was less effective in reducing the UV-B irradiation-induced wrinkling than D-methionine was.

Based on the present invention, the formulation examples comprising methionine such as a tablet, a soft capsule, a granule, beverage, a candy, a cookie, miso (soybean) paste, a French dressing, a mayonnaise, a French bread, a soy sauce, dried seasoning powder for rice, yogurt, seasoning sauce/sauce for natto (Japanese fermented soybean paste), natto, unrefined black vinegar are shown below. Methionine in the following Formulation Examples is in D-form and/or L-form. These formulation examples are listed only for the purpose of exemplification and not intended to limit the scope of the invention.

### Formulation Example 1 (Tablet)

| (Composition) | Content (mg/tablet) |
|---|---|
| Methionine | 360.5 |
| Lactose | 102.4 |
| Calcium carboxymethyl cellulose | 29.9 |
| Hydroxypropyl cellulose | 6.8 |
| Magnesium stearate | 5.2 |
| Crystalline cellulose | 10.2 |
| | 515.0 |

### Formulation Example 2 (Tablet)

| (Composition) | Content (mg/tablet) |
|---|---|
| Sucrose ester | 70 |
| Crystalline cellulose | 74 |
| Methyl cellulose | 36 |
| Glycerin | 25 |
| Methionine | 475 |
| N-Acetylglucosamine | 200 |
| Hyaluronic acid | 150 |
| Vitamin E | 30 |
| Vitamin B6 | 20 |
| Vitamin B2 | 10 |
| α-Lipoic acid | 20 |
| Coenzyme Q10 | 40 |
| Ceramide (Konjac extract) | 50 |
| L-proline | 300 |
| | 1500 |

### Formulation Example 3 (Soft capsule)

| (Composition) | Content (mg/capsule) |
|---|---|
| Edible soybean oil | 530 |
| Eucommia ulmoides extract | 50 |
| Ginseng extract | 50 |
| Methionine | 100 |
| Royal jelly | 50 |
| Maca | 30 |
| GABA | 30 |
| Beeswax | 60 |
| Gelatin | 375 |
| Glycerin | 120 |
| Glycerin fatty acid ester | 105 |
| | 1500 |

### Formulation Example 4 (Soft capsule)

| (Composition) | Content (mg/capsule) |
|---|---|
| Brown rice germ oil | 659 |
| Methionine | 500 |
| Resveratrol | 1 |
| Lotus germ extract | 100 |
| Elastin | 180 |
| DNA | 30 |
| Folic acid | 30 |
| | 1500 |

### Formulation Example 5 (Granule)

| (Composition) | Content (mg/pack) |
|---|---|
| Methionine | 400 |
| Vitamin C | 100 |
| Soybean isoflavon | 250 |
| Reduced lactose | 300 |
| Soybean oligosaccharide | 36 |
| Erythritol | 36 |
| Dextrin | 30 |
| Flavoring agent | 24 |
| Citric acid | 24 |
| | 1200 |

### Formulation Example 6 (Beverage)

| (Composition) | Content (g/60 mL) |
|---|---|
| Eucommia ulmoide extract | 1.6 |
| Carrot extract | 1.6 |
| Methionine | 1.6 |
| Reduced maltose syrup | 28 |
| Erythritol | 8 |
| Citric acid | 2 |
| Flavoring agent | 1.3 |
| N-Acetylglucosamine | 1 |
| Sodium hyaluronate | 0.5 |
| Vitamin E | 0.3 |
| Vitamin B6 | 0.2 |
| Vitamin B2 | 0.1 |
| α-Lipoic acid | 0.2 |
| Coenzyme Q10 | 1.2 |
| Ceramide (Konjac extract) | 0.4 |
| L-Proline | 2 |
| Purified water | Remainder |
| | 60 |

### Formulation Example 7 (Candy)

| (Composition) | Content (% by weight) |
|---|---|
| Sugar | 50 |
| Syrup | 48 |
| Methionine | 1 |
| Flavoring agent | 1 |
| | 100 |

### Formulation Example 8 (Cookie)

| (composition) | Content (% by weight) |
|---|---|
| Weak flour | 45.0 |
| Butter | 17.5 |
| Granulated sugar | 20.0 |
| Methionine | 4.0 |
| Egg | 12.5 |
| Flavoring agent | 1.0 |
| | 100.0 |

### Method for producing Formulation Example 8 (Cookie)

Granular sugar was added in portions to butter while stirring, to which an egg, methionine and a flavoring agent were added and stirred. After mixing thoroughly, uniformly sieved weak flour was added and then stirred slowly, and allowed to stand as a bulk in a refrigerator. Thereafter, it was molded and baked for 15 minutes at 170°C (degrees Celcius) to obtain a cookie.

### Formulation Example 9 (Miso (Soybean) paste)

| (Composition) | Content (g) |
|---|---|
| Soybean | 1000 |
| Malted rice | 1000 |
| Salt | 420 |
| Methionine | 158 |
| Water | Remainder |
| | 4000 |

### Method for producing Formulation Example 9 (Miso seasoning paste)

Malted rice is mixed thoroughly with a salt. Washed soybeans are soaked in three times its volume of water, which are then drained off, and new water is added while boiling, and poured into a colander to collect the broth (tanemizu fluid), to which methionine is dissolved at 10% w/v. The boiled beans are minced immediately, combined with malted rice mixed with salt, to which the tanemizu fluid described above containing methionine dissolved therein is added and kneaded evenly to obtain a clay-like hardness. Dumplings are made and stuffed in a container compactly without forming any void, and the surface of the content is smoothened and sealed by wrapping with a plastic film. After three months, the content is transferred to a new container and the surface is smoothened and sealed by wrapping with a plastic film. Instead of adding methionine to the tanemizu fluid, a malted rice producing a large amount of methionine may be employed. Such malted rice can be selected by quantifying methionine by the method described in Japanese Patent Unexamined Publication No. 2008-185558. Alternatively, a commercially available miso seasoning paste can be supplemented with methionine or a salt thereof.

### Formulation Example 10 (French dressing)

| (Composition) | Content (g) |
|---|---|
| Salad oil | 27.0 |
| Vinegar | 30.0 |
| Sodium chloride | 0.9 |
| Methionine | 1.1 |
| Pepper | 1.0 |
| | 60.0 |

### Method for producing Formulation Example 10 (French dressing)

Vinegar is combined with sodium chloride as well as methionine, stirred thoroughly and then a pepper is added.

### Formulation Example 11 (Mayonnaise)

| (Composition) | Content (g) |
|---|---|
| Salad oil | 134.0 |
| Vinegar | 5 |
| Sodium chloride | 0.9 |
| Methionine | 1 |
| Egg yolk | 18 |
| Sugar | 0.2 |
| Pepper | 0.9 |
| | 160.0 |

### Method for producing Formulation Example 11 (Mayonnaise)

Egg yolk is combined with vinegar, sodium chloride, methionine and pepper, and agitated thoroughly using a whipping apparatus. Stirring is continued while adding salad oil in portions to form an emulsion. Finally, a sugar is added and the mixture is stirred.

### Formulation Example 12 (French bread)

| (Composition) | Content (g) |
|---|---|
| Hard flour | 140 |
| Weak flour | 60 |
| Sodium chloride | 3 |
| Sugar | 6 |
| Methionine | 2 |
| Dry yeast | 4 |
| Lukewarm water | 128 |
| | 343 |

### Method for producing Formulation Example 12 (French bread)

Lukewarm water is combined with 1 g of sugar and dry yeast, which is then allowed to undergo a pre-fermentation. Hard flour, weak flour, sodium chloride and 5 g of sugar are placed in a bowl together with Methionine, into which the pre-fermented yeast is placed. After kneading thoroughly into a ball-like dough, a primary fermentation is conducted at 30°C(degrees Celcius). The dough is kneaded again and allowed to stand, and then shaped into suitable forms, which are subjected to a final fermentation using an electronic fermentation machine. After forming coupes, baking is conducted for 30 minutes in an oven at 220°C (degrees Celcius).

### Formulation Example 13 (Soy sauce)

| (Composition) | Content (g) |
|---|---|
| Commercially available soy sauce | 980 |
| Methionine | 20 |
| | 1000 |

### Method for producing Formulation Example 13 (Soy sauce)

Commercially available soy sauce is supplemented with methionine, and stirred thoroughly. Instead of adding methionine or a salt thereof, malted rice producing a large amount of methionine may be employed for fermenting soy sauce. Such malted rice can be selected by quantifying methionine by the method described in Japanese Patent Unexamined Publication No. 2008-185558. Alternatively, commercially available soy sauce can be supplemented with methionine or a salt thereof.

### Formulation Example 14 (Yogurt)

| (Composition) | Content (g) |
|---|---|
| Milk | 880 |
| L.bulgaricus | 50 |
| S.thermophilus | 50 |
| Methionine | 20 |
| | 1000 |

### Method for producing Formulation Example 14 (Yogurt)

Fermentation is conducted at 40°C to 45°C. Other commercially available fermentation seed organisms may be employed and Commercially avail able yogurt may be supplemented with methionine. Instead of adding methionine or a salt thereof, a seed organism producing a large amount of methionine may be employed for fermentation. Such an organism can be selected by quantifying methionine by the method described in Japanese Patent Unexamined Publication No. 2008-185558. Alternatively, commercially available yogurt can be supplemented with Methionine or a salt thereof.

### Formulation Example 15 (Dried seasoning powder for rice)

| (Composition) | Content (g) |
|---|---|
| Methionine | 50 |
| Laver | 15 |
| Sodium L-glutamate | 10 |
| Sodium chloride | 2 |
| Roasted sesame | 10 |
| Dried mackerel flakes | 10 |
| Sugar | 1 |
| Soy sauce | 2 |
| | 100 |

### Formulation Example 16 (Seasoning, Sauce for natto)

| (Composition) | Content (g) |
|---|---|
| Commercially available sauce for natto | 9 |
| Methionine | 1 |
| | 10 |

### Formulation Example 17 (Natto)

| (Composition) | Content (g) |
|---|---|
| Commercially available natto | 19.9 |
| Methionine | 0.1 |
| | 20 |

### Method for producing Formulation Example 17 (Natto)

Instead of adding methionine or a salt thereof, an organism producing a large amount of methionine may be employed for producing natto. Such an organism can be selected by quantifying methionine by the method described in Japanese Patent Unexamined Publication No. 2008-185558. Alternatively, commercially available natto can be supplemented with methionine or a salt thereof.

### Formulation Example 18 (Unrefined black vinegar)

| (Composition) | Content (g) |
|---|---|
| Commercially available unrefined black vinegar | 900 |
| Methionine | 100 |
| | 1000 |

### Method for producing Formulation Example 18 (Unrefined black vinegar)

Instead of adding methionine or a salt thereof, an organism producing a large amount of methionine may be employed for producing vinegar, black vinegar or unrefined vinegar. Such an organism can be selected by quantifying methionine by the method described in Japanese Patent Unexamined Publication No. 2008-185558. Alternatively, commercially available unrefined black vinegar can be supplemented with methionine or a salt thereof.

## Claims

1. An oral composition for alleviating an ultraviolet irradiation-induced damage comprising one or more compounds selected from a group consisting of methionine and derivatives and/or salts thereof.

2. The composition according to claim 1, wherein the methionine is in the D-form.

3. The composition according to claims 1 and 2, wherein the composition is an anti-wrinkle agent.

4. The composition according to any one of claims 1 to 3, wherein the composition is used as a pharmaceutical product for skin disease.

5. The composition according to claim 4, wherein the skin disease is selected from a group consisting of erythema, solar dermatitis, chronic actinic dermatopathy, actinic keratosis, actinic cheilitis, Favre-Racouchot disease, photodermatosis, photocontact dermatitis, berloque dermatitis, photosensitive drug eruption, polymorphous light eruption, hydroa vacciniforme, solar urticaria, chronic photosensitive dermatitis, xeroderma pigmentosum, ephelides, porphyria, pellagra, Hartnup disease, solar keratosis, dermatomyositis, lichen planus, Darier disease, pityriasis rubra pilaris, rosacea, atopic dermatitis, chloasma, prurigo simplex, lupus erythematosus, squamous cell carcinoma, basal cell carcinoma and Bowen disease.

6. The composition according to claim 4 or 5, wherein the pharmaceutical product for skin disease is an agent for treating skin disease.

7. The composition according to claim 4 or 5, wherein the pharmaceutical product for skin disease is an agent for preventing skin disease.

8. The composition according to claim 1 or 2, wherein the composition is used as a food product.

9. The composition according to claim 1 or 2, wherein the composition is used as a pharmaceutical product for cataract.

10. The composition according to claim 9, wherein the pharmaceutical product for cataract is an agent for treating or preventing cataract.
